# EUROPEAN PATENT SPECIFICATION

(11) **EP 3 780 524 B1**
(45) Date of publication and mention of the grant of the patent: **12.07.2023**
(21) Application number: 19774443.6
(22) Date of filing: 29.01.2019
(51) Int. Cl.: A61B 1/00, G06F 13/00, H04Q 9/00

(54) **PROCESSOR DEVICE FOR ENDOSCOPE, AND ENDOSCOPE DATA COLLECTION SERVER**
PROZESSORVORRICHTUNG FÜR ENDOSKOP, UND ENDOSKOPDATENSAMMELSERVER
DISPOSITIF DE PROCESSEUR POUR ENDOSCOPE, ET SERVEUR DE COLLECTE DE DONNÉES D'ENDOSCOPE

(30) Priority: 29.03.2018 JP 2018063495
(43) Date of publication of application: 17.02.2021
(73) Proprietor: FUJIFILM Corporation, Tokyo 106-8620 (JP)
(72) Inventor: MIURA Nobuyuki, Ashigarakami-gun, Kanagawa 258-8538 (JP)
(74) Representative: Klunker IP Patentanwälte PartG mbB
(86) International application number: PCT/JP2019/002932
(87) International publication number: WO 2019/187600

(56) References cited:
- JP-A- 2001 144 761
- JP-A- 2002 263 063
- JP-A- 2003 076 623
- JP-A- 2005 124 823
- JP-A- 2005 136 595
- JP-A- 2012 194 835
- US-A1- 2002 126 204
- US-A1- 2012 239 803

## Description

### BACKGROUND OF THE INVENTION

### 1. Field of the Invention

The present invention relates to an endoscope processor device, and an endoscope data collection server for collecting endoscope operation data such as instructional information related to instructions on an endoscope and the like.

### 2. Description of the Related Art

Endoscopic diagnosis, which uses an endoscope system comprising an endoscope, a light source device, and a processor device, is performed in a medical field. For example, in a case where a light source lamp, which easily deteriorates with age, such as a xenon lamp, is used in the light source device of the endoscope system, the light source lamp needs to be replaced regularly. However, since the time when maintenance is to be performed or the time when the light source lamp is to be replaced varies for each endoscope system, there are many cases where a timing when maintenance is to be performed cannot be known.

In contrast, in JP2002-263063A, a processor device for an endoscope includes a monitoring circuit for monitoring devices connected to a processor for an endoscope and is adapted to inform a server for service, which is connected to a network, of monitoring results of the monitoring circuit. Accordingly, the time when maintenance is to be performed and the like can be grasped in the server for service.

US2002126204A1 discloses an endoscope system provided with an endoscope processor that processes an image signal output by an electronic endoscope, an endoscope controlling system connected to the endoscope processor to control operation of the endoscope processor or a device connected to the endoscope processor. An endoscope server communicates with the endoscope controlling system through a first network, and a service server communicates with at least one of the endoscope processor and the device connected to the endoscope processor through a second network. A surveillance circuit surveys operation of the endoscope processor or the device connected to the endoscope processor, the endoscope controlling system transmits a surveying result to the endoscope server through the first network, and the endoscope server transmits the surveying result of the surveillance circuit to the service server through the second network. US2012239803A1 discloses a monitoring apparatus, monitoring system, and information setting method. The monitoring apparatus includes a storage and a processor. The storage stores collecting apparatus information for identifying one of a plurality of collecting apparatuses collecting status information of a network. The processor acquires status information of the network from a network apparatus connected to the network. The processor acquires, from a database, collecting apparatus information for identifying each of the plurality of collecting apparatuses. The processor acquires, based on the acquired collecting apparatus information, load information indicating a load on each of the plurality of collecting apparatuses. The processor selects, based on the acquired load information, one collecting apparatus among the plurality of collecting apparatuses, to which the monitoring apparatus transmits the acquired status information. The processor stores collecting apparatus information for identifying the selected collecting apparatus in the storage. The processor notifies the selected collecting apparatus of monitoring apparatus information for identifying the monitoring apparatus.

### SUMMARY OF THE INVENTION

Not only direct information about maintenance, such as the hours of use of a light source, but also indirect information not directly relating to maintenance, that is, endoscope operation data relating to the operation of an endoscope, such as information about ON/OFF of power, the instructional information of a mode changeover switch for switching illumination light or image processing, or the instructional information of angle knobs for bending the distal end portion of an insertion part of the endoscope, have been collected in recent years so that more efficient maintenance is performed.

It is preferable that instructional information about an instruction performed during the diagnosis of an endoscope among the endoscope operation data is also transmitted in real time. Accordingly, there is a demand for the reliable and efficient transmission of endoscope operation data to access points provided to collect endoscope operation data in a case where the reliability of the communication of endoscope operation data is ensured even though an abnormality occurs in a network or an abnormality occurs in an endoscope data collection server collecting endoscope operation data.

An object of the invention is to provide a processor device for an endoscope, and an endoscope data collection server that can ensure the reliability of the communication of endoscope operation data even though an abnormality occurs in a network or the like.

In one aspect, there is provided a processor device according to claim 1 of the appended claims.

It is preferable that the connection access point determination unit calculates connection times, which pass until the processor device for an endoscope is completely connected to the respective access points, using the connection request and the response information and determines the access point, to which the processor device for an endoscope is completely connected in a shortest time among the connection times, as the access point for constant connection.

It is preferable that endoscope data collection servers, which collect the endoscope operation data, are connected to the access points, respectively, and the endoscope data collection server for constant connection, which is always connected to the access point for constant connection, collects the endoscope operation data transmitted from the operation data transmission unit and delivers the collected endoscope operation data to the endoscope data collection servers connected to the access points other than the access point for constant connection. It is preferable that the plurality of endoscope operation data collected by the endoscope data collection servers connected to the respective access points are gathered in a specific database for an endoscope, and any one of the plurality of endoscope operation data is registered in the specific database for an endoscope.

It is preferable that the processor device for an endoscope according to the aspect of the invention further comprises a monitoring unit monitoring a communication state with the access point for constant connection and an access point change unit changing the access point for constant connection to the other access point depending on a communication state. It is preferable that a remote control device for maintenance, which is used for remote control for maintenance of an endoscope, is connected to the specific network and the processor device for an endoscope further comprises a message reception unit receiving a message for an endoscope, which is transmitted from the remote control device for maintenance, through the access point for constant connection. It is preferable that the respective access points share information about which access point is the access point for constant connection.

It is preferable that the processor device for an endoscope further comprises an installation destination information input unit inputting installation destination information and the operation data transmission unit transmits the installation destination information in addition to the endoscope operation data. It is preferable that a first installation destination information database in which identification information included in the endoscope operation data and installation destination information are stored in association with each other is connected to the specific network and the operation data transmission unit specifies the installation destination information from the identification information with reference to the first installation destination information database and transmits the installation destination information in addition to the endoscope operation data.

It is preferable that the processor device for an endoscope further comprises a positional information acquisition unit acquiring positional information, the operation data transmission unit transmits the positional information in addition to the endoscope operation data, a second installation destination information database in which the positional information and installation destination information are stored in association with each other is connected to the specific network, and an endoscope data collection server for constant connection, which is always connected to the access point for constant connection, specifies the installation destination information from the positional information, which is transmitted by the operation data transmission unit, with reference to the second installation destination information database and stores the endoscope operation data and the installation destination information such that the endoscope operation data and the installation destination information are associated with each other.

It is preferable that an IP address service server, which specifies installation destination information from an IP address of a transmission source of the operation data transmission unit, is connected to the specific network and an endoscope data collection server for constant connection, which is always connected to the access point for constant connection, stores the installation destination information specified by the IP address service server and the endoscope operation data such that the installation destination information and the endoscope operation data are associated with each other.

It is preferable that the operation data transmission unit transmits the endoscope operation data to the endoscope data collection servers, which collect the endoscope operation data, whenever the endoscope operation data is generated. It is preferable that the endoscope operation data includes instructional information about an instruction to be performed on a processor instruction unit, a scope instruction unit of an endoscope, or a light source instruction unit of a light source device, or processing information about a processor-processing unit, a scope-processing unit included in the endoscope, or a light source device-processing unit included in the light source device.

In another aspect, there is provided an endoscope data collection server according to claim 12 of the appended claims.

It is preferable that the endoscope data collection server further comprises a data delivery unit delivering the endoscope operation data collected by the data collection unit to another endoscope data collection server connected to another access point provided on the specific network. It is preferable that the data collection unit receives the endoscope operation data whenever the endoscope operation data is generated.

According to the invention, it is possible to ensure the reliability of the communication of endoscope operation data even though an abnormality occurs in a network or the like.

### BRIEF DESCRIPTION OF THE DRAWINGS

Fig. 1 is a conceptual diagram showing an endoscope system that is provided in a hospital or a clinic, a plurality of access points that are connected to the endoscope system through a specific network, and the like.
Fig. 2 is a conceptual diagram showing an endoscope, a processor device, and a light source device.
Fig. 3 is a block diagram showing the functions of the processor device.
Fig. 4 is a diagram showing that a connection request is transmitted to the plurality of access points from the processor device.
Fig. 5 is a diagram showing that the processor device receives response information from each access point.
Fig. 6 is a diagram showing an access point for constant connection that is determined among the plurality of access points.
Fig. 7 is a diagram showing that endoscope operation data collected by an endoscope data collection server connected to an access point for constant connection is delivered to endoscope data collection servers connected to the other access points.
Fig. 8 is a diagram showing that an access point for constant connection is changed depending on a communication state.
Fig. 9 is a diagram showing that a remote control device for maintenance is connected to the processor device through an access point for constant connection.
Fig. 10 is a diagram showing a method of collecting endoscope operation data in a case where installation destination information is input to the processor device.
Fig. 11 is a diagram showing a method of collecting endoscope operation data in a case where installation destination information is acquired from the identification information of the endoscope operation data.
Fig. 12 is a diagram showing a method of collecting endoscope operation data in a case where installation destination information is acquired from positional information.
Fig. 13 is a diagram showing a method of collecting endoscope operation data in a case where installation destination information is acquired from an IP address.
Fig. 14 is a block diagram showing the functions of an endoscope management system.
Fig. 15 is a diagram showing a method of calculating the next estimated failure date of an endoscope.
Fig. 16 is an image diagram of a monitor that suggests a candidate endoscope to be used next and the like.

### DESCRIPTION OF THE PREFERRED EMBODIMENTS

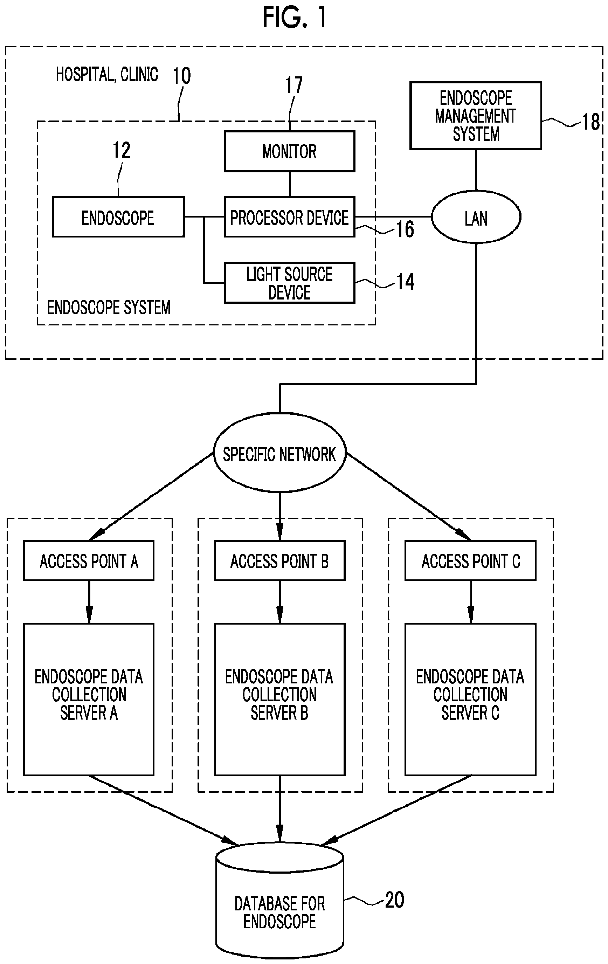

As shown in Fig. 1, an endoscope system 10 is provided for endoscopy in medical facilities, such as a hospital and a clinic. The endoscope system 10 comprises an endoscope 12 that is to be inserted into the body of a patient, a light source device 14 that supplies illumination light for illuminating the inside of the body of the patient to the endoscope 12, and a processor device 16 (a processor device for an endoscope) that performs image processing on the image of an object to be observed pickup by the endoscope 12. Further, a monitor 17, which is used to display images and the like output from the processor device 16, is connected to the processor device 16. A plurality of endoscope systems 10 are provided and a plurality of endoscopes 12 can be mounted on one processor device 16 and one light source device 14. The processor device 16 is connected to a local area network (LAN), and various data output from the processor device 16 are output through the LAN. Further, an endoscope management system 18, which manages the respective endoscopes 12 on the basis of the various data output from the processor device 16, is connected to the LAN.

The LAN of medical facilities is further connected to a specific network. For example, a closed network, which is adapted to use a mobile phone network without passing through the Internet, is used as the specific network. A plurality of access points A, B, and C are connected to the specific network as bases where endoscope operation data output from the processor device 16 are collected. The endoscope operation data includes instructional information about an instruction to be performed on a scope instruction unit 30 of the endoscope 12, a light source instruction unit 32 of the light source device 14, or a processor instruction unit 34 of the processor device 16 as described later. Further, the endoscope operation data is data including processing information about a scope-processing unit (for example, an output circuit of an image pickup element, or the like) included in the endoscope 12, a light source device-processing unit (a light source control unit for controlling a light source emitting illumination light, or the like) included in the light source device 14, or a processor-processing unit (an image processing unit for performing image processing on an image, or the like) included in the processor device 16.

In a case where a mobile phone network, which is a closed network, is used as the specific network, it is preferable that devices (for example, an Ethernet and a router (gateway) for 3G/LTE), which are to be connected to the mobile phone network, are installed on the LAN in a case where the processor device 16 is connected to the LAN as in this embodiment. Alternatively, in a case where the processor device 16 and the specific network are directly connected to each other, it is preferable that a device (a communication modem for 3G/LTE), which is to be connected to the mobile phone network is built in the processor device 16. Further, for example, a virtual private network (VPN) constructed on the Internet may be used as the specific network.

The locations of the access points A, B, and C are different from each other. For example, it is preferable that the access point A is located at a specific city in Japan, the access point B is located at a specific city in the United States of America, and the access point C is located at a specific city in Europe. Further, endoscope data collection servers A, B, and C, which are used to collect endoscope operation data, are connected to the access points A to C, respectively. The endoscope operation data collected by the endoscope data collection servers A, B, and C are gathered in a specific database 20 for an endoscope. Only any one of the endoscope operation data collected by the endoscope data collection servers A, B, and C is registered in the specific database 20 for an endoscope so that the same endoscope operation data do not duplicate (For example, in a case where endoscope operation data collected by the endoscope data collection server B are already registered in the specific database 20 for an endoscope, endoscope operation data gathered subsequently are discarded).

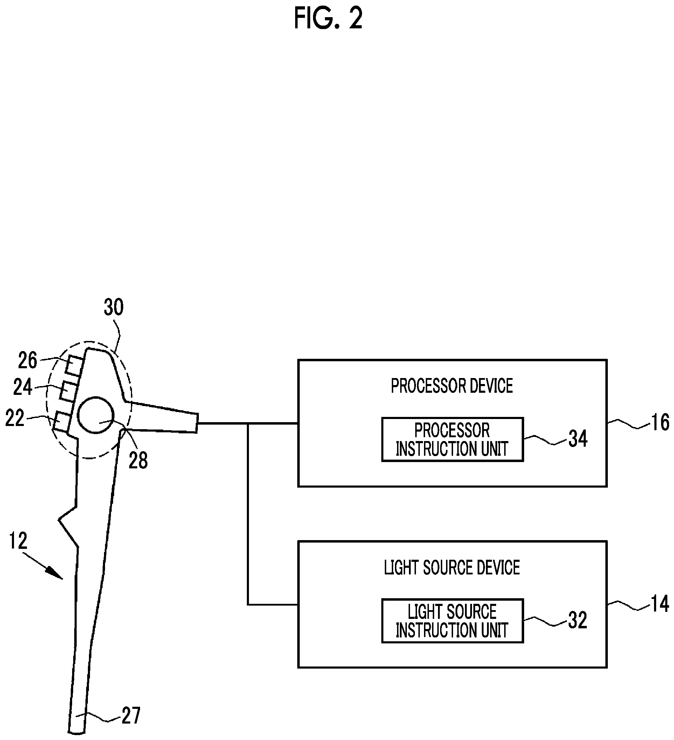

As shown in Fig. 2, the endoscope 12 is provided with a scope instruction unit 30, such as a freeze button 22 used to acquire the static image of an object to be observed, a mode changeover switch 24 used to switch the pattern of illumination light or an image display pattern, a zoom instruction part 26 used to increase or reduce the displayed size of the object to be observed, and angle knobs 28 used to change the direction of a distal end portion 27 of an insertion part. The light source device 14 is provided with a light source instruction unit 32, such as an adjustment switch used to adjust the amount of illumination light, in addition to a power switch. The processor device 16 is provided with a processor instruction unit 34 that relates to image processing and various kinds of control. The endoscope 12 is connected to each of the light source device 14 and the processor device 16, and the light source device 14 and the processor device 16 are also electrically connected to each other.

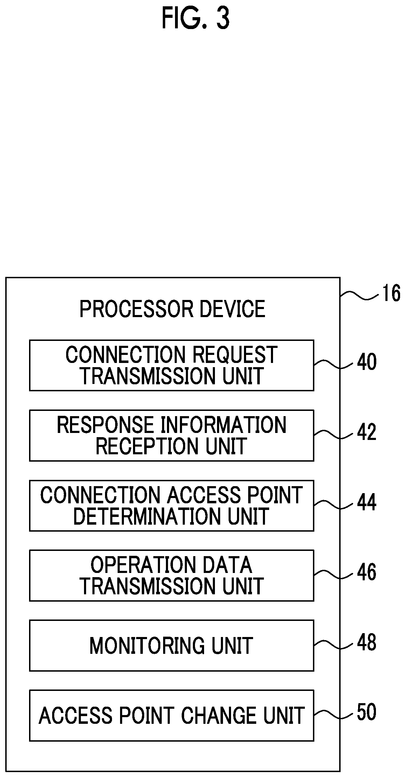

As shown in Fig. 3, the processor device 16 comprises a connection request transmission unit 40, a response information reception unit 42, a connection access point determination unit 44, and an operation data transmission unit 46 to output endoscope operation data, which are generated by the endoscope 12, the light source device 14, or the processor device 16, to the endoscope data collection servers A, B, and C. In addition, the processor device 16 comprises a monitoring unit 48 and an access point change unit 50 so that an access point can be changed depending on a communication state with the access point.

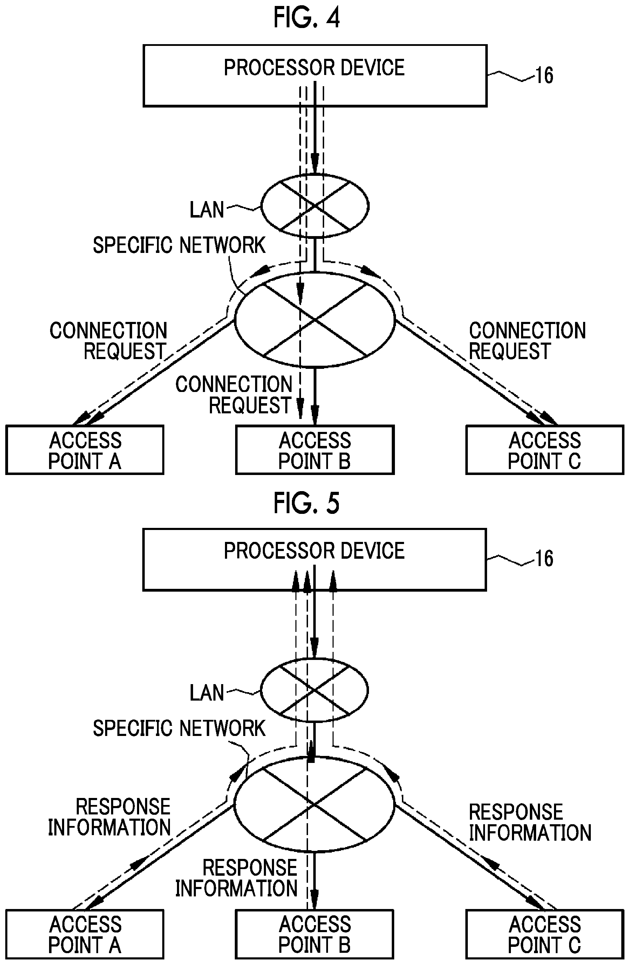

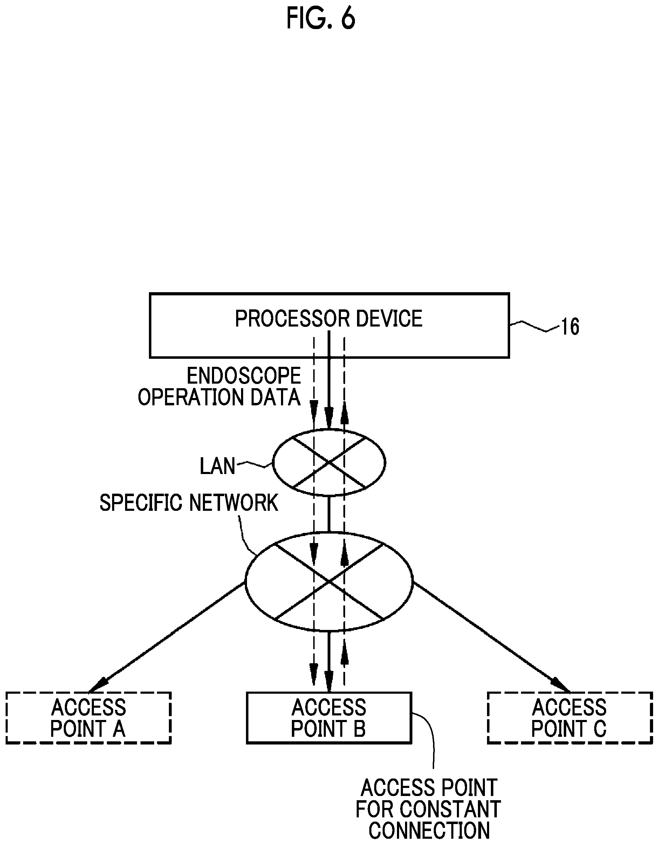

As shown in Fig. 4. the connection request transmission unit 40 transmits a connection request, which requests connection to the processor device 16, to the access points A, B, and C. In a case where the access points A, B, and C receive a connection request from the processor device 16, the access points A, B, and C output response information to the processor device 16 as shown in Fig. 5. The response information reception unit 42 receives the response information issued from the access points A, B, and C. The connection access point determination unit 44 calculates connection times A, B, and C, which are required for the connection of the respective access points A, B, and C, from a time when the connection request is transmitted and times when the response information is received from the access points A, B, and C. Then, the connection access point determination unit 44 determines an access point, which is connected in the shortest connection time among the connection times A, B, and C, as an access point for constant connection that is to be always connected. Accordingly, an access point where a load is substantially low can be selected in consideration of a network path and the congestion of the server. For example, in a case where the connection time B is shortest, the access point B is determined as an access point for constant connection as shown in Fig. 6. Constant connection means that data output from the processor device 16 is transmitted to only the access point determined for constant connection and is not transmitted to access points other than an access point for constant connection.

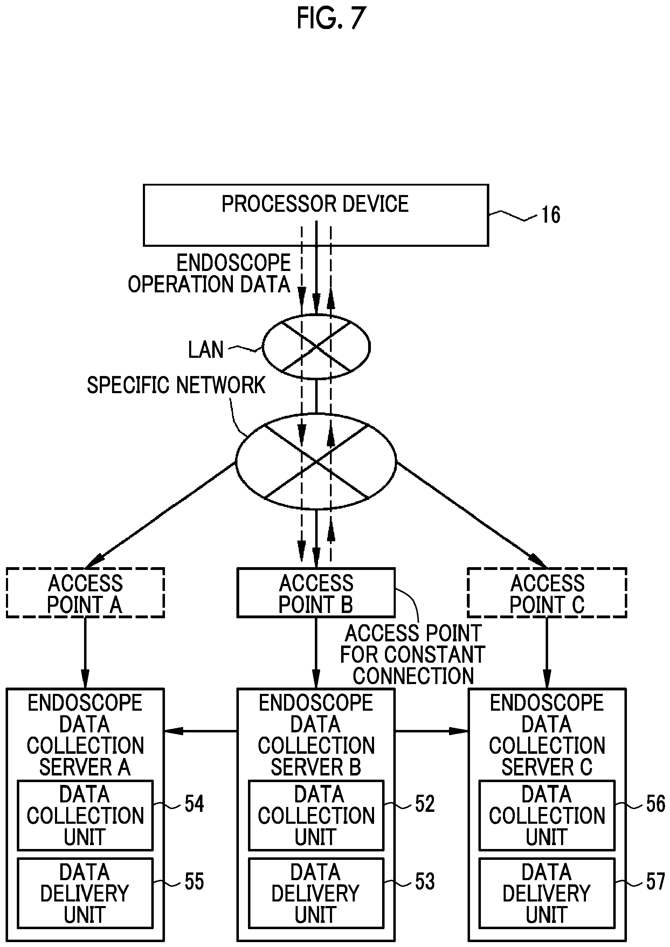

In a case where an access point for constant connection is determined, the operation data transmission unit 46 transmits endoscope operation data to the access point B, which is an access point for constant connection, whenever the endoscope operation data is generated. As shown in Fig. 7, whenever endoscope operation data is generated, the endoscope operation data is collected at the access point B by a data collection unit 52 of the endoscope data collection server B. Further, the endoscope data collection server B is connected to the other endoscope data collection servers A and C, and a data delivery unit 53 of the endoscope data collection server B delivers the received endoscope operation data to the other endoscope data collection servers A and C. Accordingly, since data is backed up in the other endoscope data collection servers A and C, even if a trouble occurs at the access point B or in the endoscope data collection server B, the loss of endoscope operation data can be prevented. The endoscope data collection server A is also provided with a data collection unit 54 and a data delivery unit 55 that are the same as the data collection unit 52 and the data delivery unit 53 of the endoscope data collection server B, and the endoscope data collection server C is also provided with a data collection unit 56 and a data delivery unit 57 that are the same as the data collection unit 52 and the data delivery unit 53 of the endoscope data collection server B.

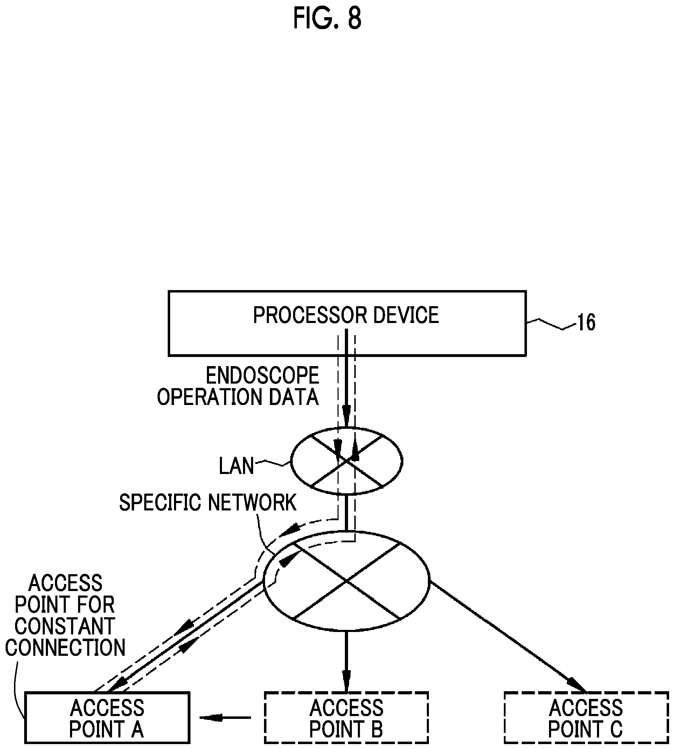

Furthermore, the monitoring unit 48, which monitors a communication state with an access point for constant connection, is provided in the processor device 16. The monitoring unit 48 monitors the transmission time of the endoscope operation data transmitted to the access point B that is an access point for constant connection. The transmission time of the endoscope operation data is calculated from a time when the endoscope operation data is transmitted and a time when the response information issued from the access point B receiving the endoscope operation data is received by the response information reception unit 42. In a case where the monitoring unit 48 determines that the transmission time of the endoscope operation data exceeds a specific threshold value, the access point change unit 50 changes an access point for constant connection to the other access point A or C from the access point B as shown in Fig. 8 (in Fig. 8, the access point A is changed to an access point for constant connection). Which one of the access points A and C is set as an access point for constant connection is determined using the connection request and the response information as described above.

Further, a remote control device 60 for maintenance, which is used for the remote control for maintenance of the endoscope, is connected to the network. The remote control device 60 for maintenance transmits a message for an endoscope, which shows a warning or the like about the control of an endoscope, to the processor device 16 as one of the remote control for maintenance. However, the remote control device 60 for maintenance does not include information about which one of the access points A, B, and C that the processor device 16 corresponding to the transmission destination of the message for an endoscope is connected to. For this reason, information about which access point the processor that device 16 is constantly connected to is shared among the respective access points A, B, and C.

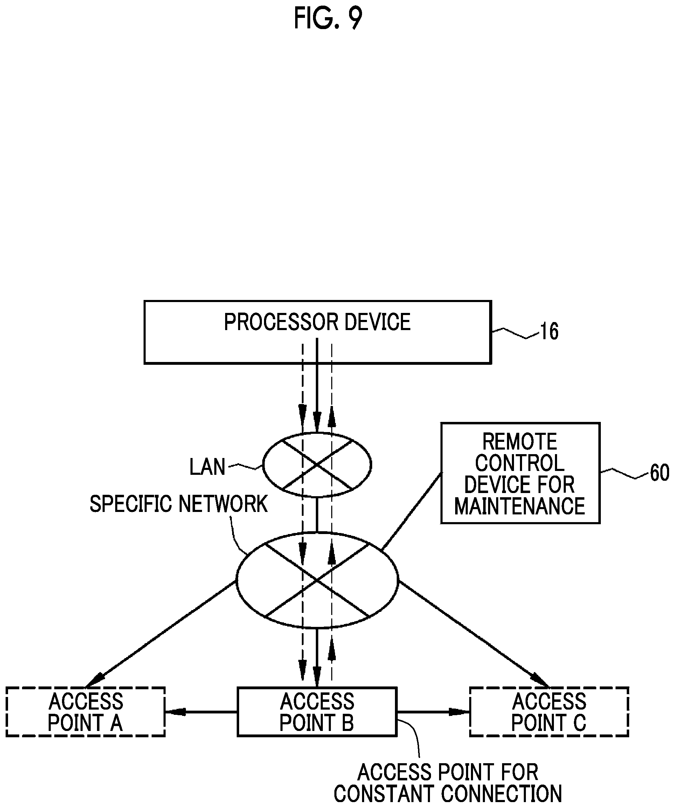

Accordingly, even in a case where the remote control device 60 for maintenance is connected to any one of the access point A, B, or C, an access point (access point B) constantly connected to the processor device 16 can be grasped as shown in Fig. 9. Therefore, the remote control device 60 for maintenance can reliably transmit the message for an endoscope to the processor device 16 that is an object to which a message is to be transmitted. The transmitted message for an endoscope is received by a message reception unit provided in the processor device 16. The received message for an endoscope is displayed on a monitor connected to the processor device 16 and is thus used for maintenance and the like performed by a serviceman.

Information specifying a client is not included in the processor device 16 at the time of manufacture. For this reason, since installation destination information representing the installation location of the processor device is not included in the endoscope operation data transmitted from the processor device 16, it is not possible to specify which client uses the endoscope operation data. The lack of this installation destination information causes problems in that it is difficult to analyze and predict data on the basis of the endoscope operation data and an active action cannot be taken even though maintenance becomes necessary. For this reason, the endoscope operation data is adapted to specify installation destination information as described below so that a client from which the endoscope operation data is transmitted is grasped.

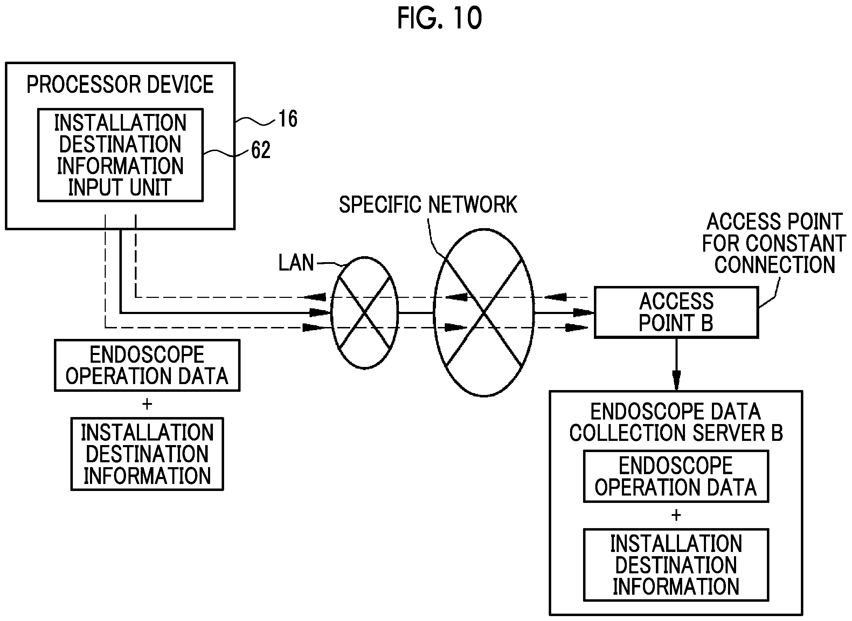

For example, the operation data transmission unit 46 may be adapted to transmit installation destination information, which is the installation location of the endoscope system 10 (or the processor device 16), in addition to the endoscope operation data in a case where the operation data transmission unit 46 of the processor device 16 transmits endoscope operation data. In this case, the processor device 16 is provided with an installation destination information input unit 62 used to input installation destination information. In a case where installation destination information (for example, "Hospital A") is input by the installation destination information input unit 62. the installation destination information is generated. As shown in Fig. 10, the generated installation destination information is transmitted together in a case where the endoscope operation data is transmitted. The endoscope operation data and the installation destination information are stored in the endoscope data collection server (in Fig. 10, the endoscope data collection server B that is an access point for constant connection), which receives the installation destination information and the endoscope operation data, in association with each other.

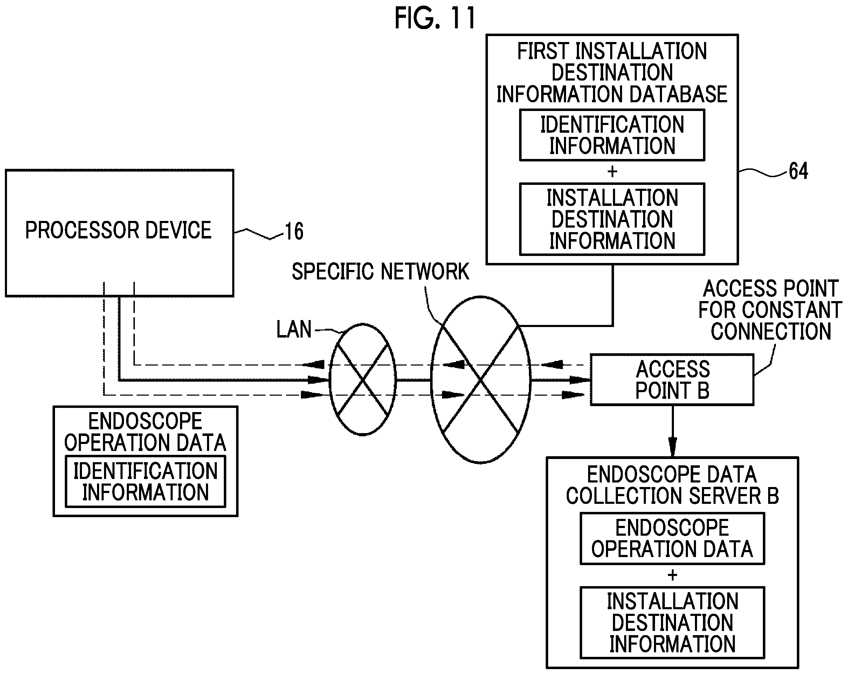

Further, in a case where identification information (for example, a model number and/or a manufacture serial number) for identifying the processor device 16 (or the endoscope system 10) is included in the endoscope operation data as shown in Fig. 11, the installation destination information may be specified from the identification information included in the endoscope operation data with reference to a first installation destination information database in which the identification information and the installation destination information are stored in association with each other. Since the first installation destination information database is connected to the specific network, the operation data transmission unit 46 specifies the installation destination information (for example, "Hospital A") from the identification information included in the endoscope operation data with reference to the first installation destination information database 64 in a case where the endoscope operation data is transmitted. Then, the operation data transmission unit 46 transmits the specified installation destination information together with the endoscope operation data. Similarly to the above, the endoscope operation data and the installation destination information are stored in the endoscope data collection server (in Fig. 11, the endoscope data collection server B that is an access point for constant connection), which receives the installation destination information and the endoscope operation data, in association with each other.

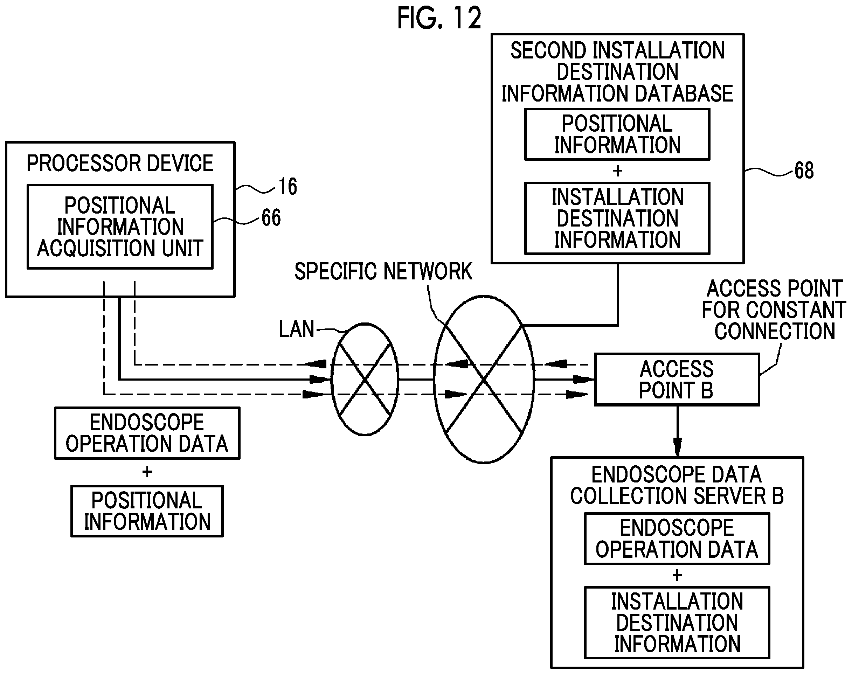

Furthermore, in a case where a positional information acquisition unit 66 for acquiring the positional information of a global pointing system (GPS), a WiFi-access point (WiFi-AP), a mobile phone base station, and the like is provided in the processor device 16, the operation data transmission unit 46 transmits positional information together with the endoscope operation data as shown in Fig. 12. The endoscope data collection server (in Fig. 12, the endoscope data collection server B that is an access point for constant connection), which receives the positional information and the endoscope operation data, can be connected to a second installation destination information database 68. in which the positional information and the installation destination information are stored in association with each other. through the specific network or the like. The endoscope data collection server specifies the installation destination information from the positional information with reference to the second installation destination information database 68. The specified installation destination information and the endoscope operation data are recorded in the endoscope data collection server in association with each other.

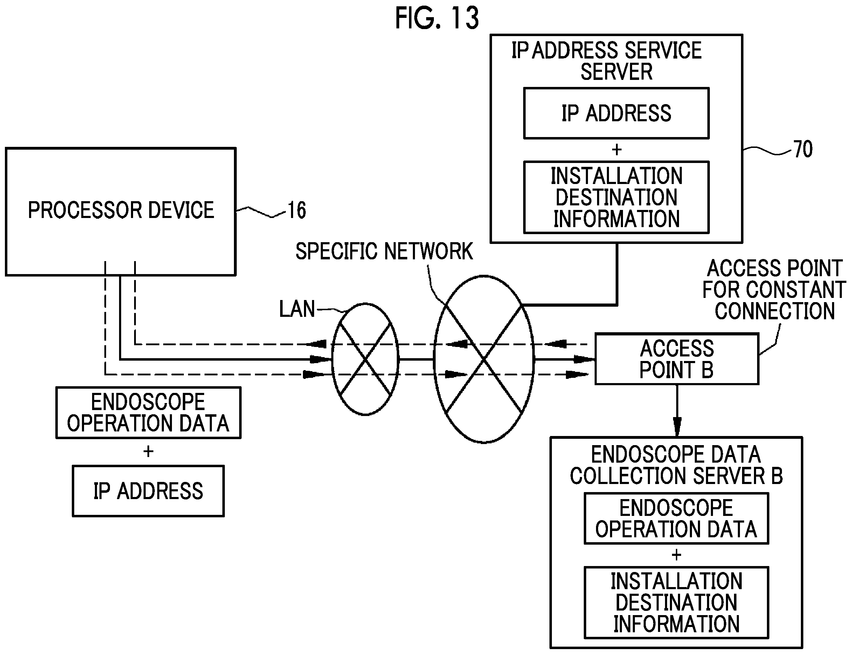

Further, in a case where the endoscope operation data is transmitted through the specific network and the IP address of the processor device 16 serving as a transmission source is disclosed as in this embodiment, the installation destination information may be acquired from the IP address as shown in Fig. 13. In this case, the endoscope data collection server (in Fig. 13, the endoscope data collection server B that is an access point for constant connection), can be connected to an IP address service server 70, which provides the installation destination information from the IP address, through the specific network or the like. The IP address service server 70 specifies the installation destination information from the IP address attached to the endoscope operation data. The specified installation destination information is stored in the endoscope data collection server in association with the endoscope operation data.

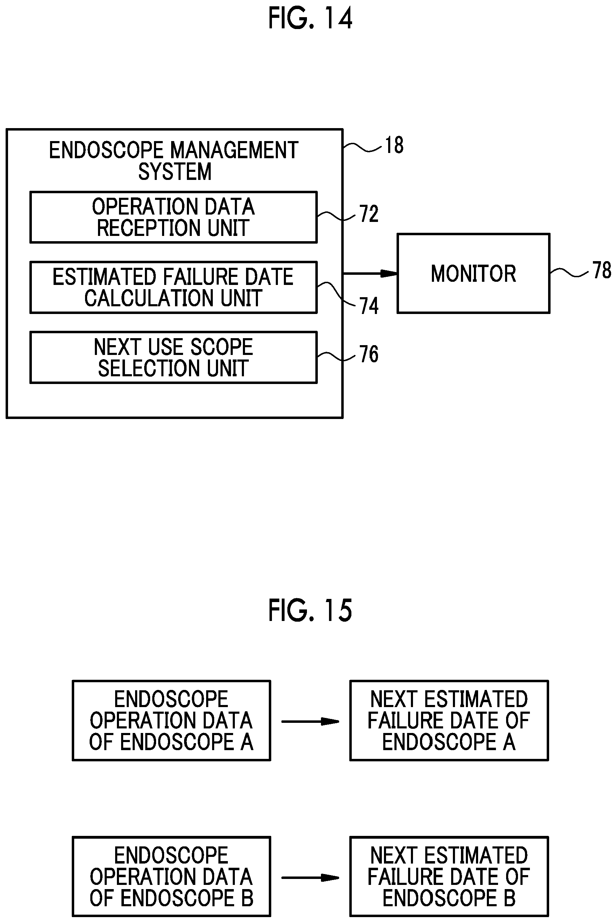

As shown in Fig. 14. the endoscope management system 18 comprises an operation data reception unit 72 that receives the endoscope operation data, an estimated failure date calculation unit 74 (use state calculation unit) that calculates the next estimated failure date of the respective endoscopes 12 as the use states of the respective endoscopes 12 on the basis of the endoscope operation data about the respective endoscopes 12, and a next use scope selection unit 76 that selects a candidate endoscope 12 to be used next, which is an object to be used next, among the plurality of endoscopes 12 using the next estimated failure dates of the respective endoscopes 12. The operation data reception unit 72 receives endoscope data that is transmitted in a case where the endoscope 12 is mounted on the processor device 16. The operation data transmission unit 46 of the processor device 16 transmits the endoscope operation data to an access point for constant connection, but transmits the endoscope operation data to not only an access point for constant connection but also the endoscope management system 18 in a case where the endoscope 12 is mounted on the processor device 16.

As shown in Fig. 15, the estimated failure date calculation unit 74 calculates the next estimated failure date of an endoscope A from the endoscope operation data of the endoscope A. It is preferable that the next estimated failure date is determined according to. for example, the number of uses of the angle knobs 28 among the endoscope operation data in a method of calculating the next estimated failure date. In this case, it is preferable that the next estimated failure date is advanced as the number of uses of the angle knobs is increased. Likewise, the estimated failure date calculation unit 74 calculates the next estimated failure date of an endoscope B from the endoscope operation data of the endoscope B.

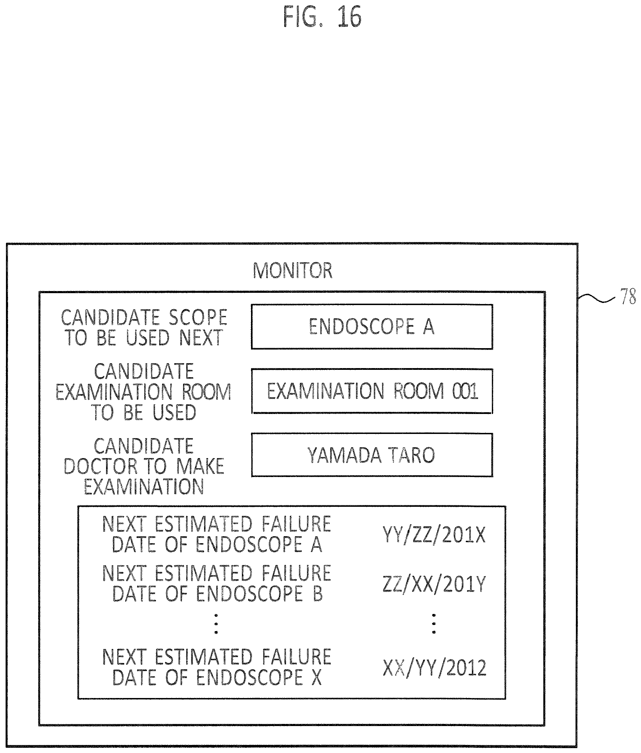

The next use scope selection unit 76 selects a candidate endoscope 12 to be used next, which is an object to be used next, among a plurality of endoscopes 12 on the basis of the next estimated failure date that is calculated by the estimated failure date calculation unit 74. For example, as shown in Fig. 16, an endoscope 12 (in Fig. 16, "endoscope A") of which the next estimated failure date is farthest from the current date is displayed on a monitor 78, which is connected to the endoscope management system 18, as a candidate endoscope 12 to be used next. Since the endoscope 12 that is unlikely to cause a failure is used in this way, time to pass until the next estimated failure can be leveled.

The monitor 78 may be caused to display a candidate examination room to be used in which an examination is made using the endoscope 12 or a candidate doctor who is to make an examination to make a diagnosis using the endoscope 12 other than a candidate endoscope 12 to be used next. Further, a candidate endoscope 12 to be used next may be suggested using a failure prediction algorithm that prevents the timings, when the failures of a plurality of endoscopes 12 occur, from being the same, in addition to a case where the endoscope 12 of which the next estimated failure date is farthest from the current date is displayed as a candidate endoscope 12 to be used next. Furthermore, a candidate endoscope 12 to be used next may be suggested so that the average time when the endoscope 12 is collected at a repair center is also included instead of or in addition to the next estimated failure date and be leveled.

In the embodiment, the hardware structures of processing units for performing various kinds of processing, that is, the respective processing units of the processor device 16, such as the connection request transmission unit 40, the response information reception unit 42, the connection access point determination unit 44, the operation data transmission unit 46. the monitoring unit 48, the access point change unit 50, the installation destination information input unit 62, and the positional information acquisition unit 66, or the processing units included in the endoscope management system 18, such as operation data reception unit 72, the estimated failure date calculation unit 74, and the next use scope selection unit 76, are various processors to be described below. The various processors include: a central processing unit (CPU) that is a general-purpose processor functioning as various processing units by executing software (program); a programmable logic device (PLD) that is a processor of which circuit configuration can be changed after manufacture, such as a field programmable gate array (FPGA): a dedicated electrical circuit that is a processor having circuit configuration designed exclusively to perform various kinds of processing; and the like.

One processing unit may be formed of one of these various processors, or may be formed of a combination of two or more same kind or different kinds of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

The invention has been applied to the processor device for an endoscope, but can also be applied to other medical devices. For example, the invention can be applied to in vitro diagnostics (IVD) that can be connected to a specific network. In this case, measurement results obtained from the in vitro diagnostics are transmitted to a measurement result collection server for constant connection through an access point for constant connection.

### Explanation of References

- 10:: endoscope system
- 12:: endoscope
- 14:: light source device
- 16:: processor device
- 17:: monitor
- 18:: endoscope management system
- 20:: database for endoscope
- 22:: freeze button
- 26:: zoom instruction part
- 27:: distal end portion
- 28:: angle knob
- 30:: scope instruction unit
- 32:: light source instruction unit
- 34:: processor instruction unit
- 40:: connection request transmission unit
- 42:: response information reception unit
- 44:: connection access point determination unit
- 46:: operation data transmission unit
- 48:: monitoring unit
- 50:: access point change unit
- 52:: data collection unit
- 53:: data delivery unit
- 54:: data collection unit
- 55:: data delivery unit
- 56:: data collection unit
- 57:: data delivery unit
- 60:: remote control device for maintenance
- 62:: installation destination information input unit
- 64:: first installation destination information database
- 66:: positional information acquisition unit
- 68:: second installation destination information database
- 70:: address service server
- 72:: operation data reception unit
- 74:: estimated failure date calculation unit
- 76:: next use scope selection unit
- 78:: monitor

## Claims

1. A processor device (16) for an endoscope (12) that is capable of being connected to a plurality of access points, which are provided for collection of endoscope operation data or remote control for maintenance, through a specific network, wherein endoscope data collection servers, which collect the endoscope operation data, are connected to the access points, respectively, the processor device (16) for an endoscope (12) comprising:
a connection request transmission unit (40) configured to transmit a connection request to the plurality of access points;
a response information reception unit (42) configured to receive response information issued from the respective access points in response to the connection request;
a connection access point determination unit (44) configured to determine an access point for constant connection, which is to be always connected, among the plurality of access points on the basis of the response information; and
an operation data transmission unit (46) configured to transmit the endoscope operation data to the access point for constant connection, wherein the endoscope operation data includes instructional information about an instruction to be performed on a processor instruction unit (34), a scope instruction unit (30) of an endoscope (12), or a light source instruction unit (32) of a light source device (14), or processing information about a processor-processing unit, a scope-processing unit included in the endoscope (12), or a light source device-processing unit included in the light source device (14);
wherein the endoscope data collection server for constant connection, which is always connected to the access point for constant connection, is configured to collect the endoscope operation data transmitted from the operation data transmission unit (46) and and to deliver the collected endoscope operation data to the endoscope data collection servers that are connected to the access points other than the access point for constant connection.

2. The processor device (16) for an endoscope (12) according to claim 1,
wherein the connection access point determination unit (44) is configured to calculate connection times, which pass until the processor device (16) for an endoscope (12) is completely connected to the respective access points, using the connection request and the response information and and to determine the access point, to which the processor device (16) for an endoscope (12) is completely connected in a shortest time among the connection times, as the access point for constant connection.

3. The processor device (16) for an endoscope (12) according to claim 1 or 2,
wherein the plurality of endoscope operation data collected by the endoscope data collection servers connected to the respective access points are gathered in a specific database (20) for an endoscope (12), and any one of the plurality of endoscope operation data is registered in the specific database (20) for an endoscope (12).

4. The processor device (16) for an endoscope (12) according to any one of claims 1 to 3, further comprising:
a monitoring unit (48) configured to monitor a communication state with the access point for constant connection; and
an access point change unit (50) configured to change the access point for constant connection to the other access point depending on a communication state.

5. The processor device (16) for an endoscope (12) according to any one of claims 1 to 4,
wherein a remote control device for maintenance (60), which is used for remote control for maintenance of an endoscope (12), is connected to the specific network, and
the processor device (16) for an endoscope (12) further comprises a message reception unit configured to receive a message for an endoscope (12), which is transmitted from the remote control device for maintenance (60), through the access point for constant connection.

6. The processor device (16) for an endoscope (12) according to any one of claims 1 to 5, wherein the respective access points are configured to share information about which access point is the access point for constant connection.

7. The processor device (16) for an endoscope (12) according to claim 1, further comprising:
an installation destination information input unit (62) configured to input installation destination information,
wherein the operation data transmission unit (46) is configured to transmit the installation destination information in addition to the endoscope operation data.

8. The processor device (16) for an endoscope (12) according to claim 1,
wherein a first installation destination information database (64) in which identification information included in the endoscope operation data and installation destination information are stored in association with each other is connected to the specific network, and
the operation data transmission unit (46) is configured to specify the installation destination information from the identification information with reference to the first installation destination information database (64), and to transmit the installation destination information in addition to the endoscope operation data.

9. The processor device (16) for an endoscope (12) according to claim 1, further comprising:
a positional information acquisition unit (66) configured to acquire positional information,
wherein the operation data transmission unit (46) is configured to transmit the positional information in addition to the endoscope operation data,
a second installation destination information database (68) in which the positional information and installation destination information are stored in association with each other is connected to the specific network, and
an endoscope data collection server for constant connection, which is always connected to the access point for constant connection, is configured to specify the installation destination information from the positional information, which is transmitted by the operation data transmission unit (46), with reference to the second installation destination information database, and to store
the endoscope operation data and the installation destination information such that the endoscope operation data and the installation destination information are associated with each other.

10. The processor device (16) for an endoscope (12) according to claim 1,
wherein an IP address service server, which is configured to specify installation destination information from an IP address of a transmission source of the operation data transmission unit (46), is connected to the specific network, and
an endoscope data collection server for constant connection, which is always connected to the access point for constant connection, is configured to store the installation destination information specified by the IP address service server and the endoscope operation data such that the installation destination information and the endoscope operation data are associated with each other.

11. The processor device (16) for an endoscope (12) according to claim 1,
wherein the operation data transmission unit (46) is configured to transmit the endoscope operation data to the endoscope data collection servers, which are configured to collect the endoscope operation data, whenever the endoscope operation data is generated.

12. An endoscope data collection server that is connectable to an access point connected to a specific network and is configured to collect endoscope operation data output from a processor device (16) for an endoscope (12), the endoscope data collection server comprising:
a data collection unit configured to collect the endoscope operation data output from the processor device (16) for an endoscope (12), wherein the endoscope operation data includes instructional information about an instruction to be performed on a processor instruction unit (34), a scope instruction unit (30) of an endoscope (12), or a light source instruction unit (32) of a light source device (14), or processing information about a processor-processing unit, a scope-processing unit included in the endoscope (12), or a light source device-processing unit included in the light source device (14);
wherein the data collection unit is configured to collect the endoscope operation data through the access point in a case where the access point is determined as an access point for constant connection, which is always connected to the processor device (16) for an endoscope, by the processor device (16) for an endoscope (12);
wherein the endoscope data collection server is configured to send response information issued via the respective access point in response to the connection request;
wherein the access point is determined to be the access point for constant connection, which is to be always connected, among a plurality of access points to which the processor device (16) is capable of being connected through a specific network, on the basis of the response information; and
wherein the endoscope data collection server, which is the endoscope data collection server for constant connection, and which is always connected to the access point for constant connection, is configured to deliver the collected endoscope operation data to the endoscope data collection servers that are connected to the access points other than the access point for constant connection.

13. The endoscope data collection server according to claim 12, further comprising:
a data delivery unit configured to deliver the endoscope operation data collected by the data collection unit to another endoscope data collection server connected to another access point provided on the specific network.

14. The endoscope data collection server according to claim 12 or 13, wherein the data collection unit is configured to receive the endoscope operation data whenever the endoscope operation data is generated.

## Patentansprüche

1. Endoskopvorrichtung (10), umfassend:
eine Hilfsmesslicht-Emissionseinheit (30), konfiguriert zum Emittieren von Hilfsmesslicht als planares Licht, das mindestens zwei erste Merkmalslinien enthält;
ein Bildgebungselement (32), konfiguriert zum Abbilden eines mit dem Hilfsmesslicht beleuchteten Subjekts;
eine Bilderfassungseinheit, konfiguriert zum Erfassen eines aufgenommenen Bilds, erhalten für den Fall, dass das Subjekt von dem Bildgebungselement abgebildet wurde, wobei das aufgenommene Bild eine an dem Subjekt ausgebildete Schnittkurve und mindestens zwei erste Merkmalspunkte, gebildet an Stellen entsprechend den beiden ersten Merkmalslinien auf der Schnittkurve enthält;
eine Positionsspezifiziereinheit (50), konfiguriert zum Spezifizieren von mindestens Positionen der beiden ersten Merkmalspunkte auf der Grundlage des aufgenommenen Bilds durch automatisches Erkennen der Positionen der zwei ersten Merkmalspunkte,
eine Messinformations-Verarbeitungseinheit (52), konfiguriert zum Berechnen von Messinformation aus den automatisch erkannten Positionen der zwei ersten Merkmalspunkte, und
eine Anzeigesteuereinheit (40), konfiguriert zum Anzeigen der Messinformation, welche die aktuelle Größe des Subjekts in dem aufgenommenen Bild repräsentiert, wozu die Positionen der zwei ersten Merkmalspunkte herangezogen werden.

2. Endoskopvorrichtung nach Anspruch 1,
bei der die Messinformation eine erste geradlinige Distanz zwischen den zwei ersten Merkmalspunkten enthält.

3. Endoskopvorrichtung nach Anspruch 1 oder 2,
bei der die Messinformation eine zweite geradlinige Distanz zwischen einem der beiden Merkmalspunkte und einen spezifischen Punkt verschieden von dem anderen der zwei ersten Merkmalspunkte enthält.

4. Endoskopvorrichtung nach einem der Ansprüche 1 bis 3,
bei der die Messinformation eine Länge eines spezifischen Schnittkurvenabschnitts der Schnittkurve zwischen den zwei ersten Merkmalspunkten enthält,
die Positionsspezifiziereinheit (50) konfiguriert ist zum Spezifizieren einer Position des spezifischen Schnittkurvenabschnitts, und
die Anzeigesteuereinheit (40) konfiguriert ist zum Anzeigen der Länge des spezifischen Schnittkurvenabschnitts in dem aufgenommenen Bild unter Verwendung der Positionen der zwei ersten Merkmalspunkte und der Position des spezifischen Schnittkurvenabschnitts.

5. Endoskopvorrichtung nach Anspruch 4,
bei der das Hilfsmesslicht eine Mehrzahl zweiter Merkmalslinien enthält, die verschieden von den ersten Merkmalslinien sind und sich zwischen letzteren befinden,
der spezifische Schnittkurvenabschnitt eine Mehrzahl zweiter Merkmalspunkte enthält, die auf der Schnittkurve durch die zweiten Merkmalslinien gebildet sind, sodass sie kleiner als die ersten Merkmalspunkte sind, und
die Positionsspezifiziereinheit (50) konfiguriert ist zum Spezifizieren der Position des spezifischen Schnittkurvenabschnitts aus der Mehrzahl zweiter Merkmalspunkte, die in dem aufgenommenen Bild enthalten sind.

6. Endoskopvorrichtung nach Anspruch 1, weiterhin umfassend:
eine Messinformations-Umschalteinheit, konfiguriert zum Umschalten der für die Anzeige vorgesehenen Messinformation in dem aufgenommenen Bild zu irgendeinem einer Mehrzahl von Stücken von Messinformation oder einer Kombination aus zwei oder mehr von einer Mehrzahl von Stücken von Messinformation dann, wenn es eine Mehrzahl von Messinformationsstücken gibt.

7. Endoskopvorrichtung nach einem der Ansprüche 1 bis 6, weiterhin umfassend:
eine Stehbilderfassungs-Befehlseinheit, konfiguriert zum Geben eines Stehbilderfassungs-Befehls zum Erfassen eines Stehbilds des aufgenommenen Bilds,
wobei die Messinformation ebenfalls zusammengespeichert wird, falls der Stehbilderfassungs-Befehl gegeben wird.

8. Endoskopvorrichtung nach einem der Ansprüche 1 bis 7, weiterhin umfassend:
eine erste Lichtquelleneinheit, konfiguriert zum Emittieren von Beleuchtungslicht zum Beleuchten des Subjekts,
wobei die Hilfsmesslicht-Emissionseinheit (30) eine zweite Lichtquelleneinheit enthält, die unabhängig von der ersten Lichtquelleneinheit vorgesehen ist, ferner ein Hilfsmess-Optikelement, konfiguriert zur Verwendung beim Erlangen des Hilfsmesslichts aus dem von der zweiten Lichtquelleneinheit emittierten Licht.

9. Endoskopvorrichtung nach Anspruch 8,
bei der die Hilfsmesslicht-Emissionseinheit (30) ein spezifisches optisches Element enthält, welches dazu dient, das Hilfsmesslicht in Richtung des Subjekts in einem Zustand zu emittieren, in welchem eine optische Achse des Bildgebungselements und eine optische Achse des Hilfsmesslichts einander kreuzen.

10. Endoskopvorrichtung nach Anspruch 9,
bei der das spezifische optische Element mit einem Antireflexionsteil ausgestattet ist.

11. Endoskopvorrichtung nach Anspruch 8,
bei der die Hilfsmesslicht-Emissionseinheit (30) einen Hilfsmessschlitz enthält, der dazu dient, das Hilfsmesslicht in Richtung des Subjekts in einem Zustand zu emittieren, in welchem eine optische Achse des Bildgebungselement und eine optische Achse des Hilfsmesslichts einander kreuzen.

12. Endoskopvorrichtung nach einem der Ansprüche 8 bis 11,
bei der die zweite Lichtquelle eine Laserlichtquelle ist.

13. Endoskopvorrichtung nach Anspruch 12,
bei der eine Wellenlänge von aus der zweiten Lichtquelle emittiertem Licht im Bereich von 495 nm bis 570 nm liegt.

14. Endoskopvorrichtung

## Revendications

1. Appareil formant endoscope (10), comprenant :
une unité d'émission de lumière de mesure auxiliaire (30), configurée pour émettre une lumière de mesure auxiliaire comme lumière planaire incluant au moins deux premières lignes de caractéristique ;
un élément d'imagerie (32) configuré pour imager un sujet éclairé avec la lumière de mesure auxiliaire ;
une unité d'acquisition d'image configurée pour acquérir une image prise obtenue dans un cas où le sujet est imagé par l'élément d'imagerie, l'image prise incluant une courbe d'intersection formée sur le sujet et au moins deux points de caractéristique formés sur des positions correspondant aux deux premières lignes de caractéristique sur la courbe d'intersection ;
une unité de spécification de position (50) configurée pour spécifier au moins des positions des deux premiers points de caractéristique sur la base de l'image prise en reconnaissant automatiquement les positions des deux premiers points de caractéristique ;
une unité de traitement d'informations de mesure (52) configurée pour calculer des informations de mesure à partir de positions reconnues automatiquement des deux premiers points de caractéristique, et
une unité de commande d'affichage (40) configurée pour afficher les informations de mesure représentant une taille réelle du sujet dans l'image prise à l'aide des positions des deux premiers points de caractéristique.

2. Appareil formant endoscope selon la revendication 1,
dans lequel les informations de mesure incluent une première distance rectiligne entre les deux points de caractéristique.

3. Appareil formant endoscope selon la revendication 1 ou 2,
dans lequel les informations de mesure incluent une seconde distance rectiligne entre un des deux points de caractéristique et un point spécifique autre que les deux premiers points de caractéristique.

4. Appareil formant endoscope selon l'une quelconque des revendications 1 à 3,
dans lequel les informations de mesure incluent une longueur d'une portion incurvée intersection spécifique de la courbe d'intersection positionnée entre les deux points de caractéristique ;
l'unité de spécification de position (50) configurée pour spécifier une position de la portion incurvée d'intersection spécifique, et
l'unité de commande d'affichage (40) configurée pour afficher la longueur de la portion incurvée d'intersection spécifique dans l'image prise à l'aide des positions des deux premiers points de caractéristique et de la position de la portion incurvée d'intersection spécifique.

5. Appareil formant endoscope selon la revendication 4,
dans lequel la lumière de mesure auxiliaire inclut une pluralité de secondes lignes de caractéristique, lesquelles sont différentes des premières lignes de caractéristique, entre les deux premières lignes de caractéristique ;
la portion incurvée d'intersection spécifique inclut une pluralité de seconds points de caractéristique, lesquels sont formés sur la courbe d'intersection par les secondes lignes de caractéristique de manière à être plus petits que les premiers points de caractéristique, et
l'unité de spécification de position (50) configurée pour spécifier la position de la portion incurvée d'intersection spécifique à partir de la pluralité de seconds points de caractéristique inclus dans l'image prise.

6. Appareil formant endoscope selon la revendication 1, comprenant en outre :
une unité de commutation d'informations de mesure configurée pour commuter les informations de mesure à afficher dans l'image prise sur l'une quelconque d'une pluralité d'éléments d'informations de mesure ou une combinaison de deux éléments ou plus parmi une pluralité d'éléments d'informations de mesure dans un cas où il existe une pluralité d'éléments d'informations de mesure.

7. Appareil formant endoscope selon l'une quelconque des revendications 1 à 6, comprenant en outre :
une unité d'instruction d'acquisition d'image statique configurée pour donner une instruction d'acquisition d'image statique pour acquérir une image statique de l'image prise,
dans lequel les informations de mesure sont également stockées ensemble dans un cas où l'instruction d'acquisition d'image statique est donnée.

8. Appareil formant endoscope selon l'une quelconque des revendications 1 à 7, comprenant en outre :
une première unité de source de lumière configurée pour émettre une lumière d'éclairage pour éclairer le sujet,
dans lequel l'unité d'émission de lumière de mesure auxiliaire (30) inclut une seconde source de lumière, laquelle est prévue indépendamment de la première source de lumière, et un élément optique de mesure auxiliaire configuré pour être utilisé afin d'obtenir la lumière de mesure auxiliaire à partir de la lumière émise à partir de la seconde unité de source de lumière.

9. Appareil formant endoscope selon la revendication 8,
dans lequel l'unité d'émission de lumière de mesure auxiliaire (30) inclut un élément optique spécifique, lequel est utilisé pour émettre la lumière de mesure auxiliaire vers le sujet dans un état où un axe optique de l'élément d'imagerie et un axe optique de la lumière de mesure auxiliaire se croisent entre eux.

10. Appareil formant endoscope selon la revendication 9,
dans lequel l'élément optique spécifique est doté d'une portion antireflet.

11. Appareil formant endoscope selon la revendication 8,
dans lequel l'unité d'émission de lumière de mesure auxiliaire (30) inclut une fente de mesure auxiliaire, laquelle est utilisée pour émettre la lumière de mesure auxiliaire vers le sujet dans un état où un axe optique de l'élément d'imagerie et un axe optique de la lumière de mesure auxiliaire se croisent entre eux.

12. Appareil formant endoscope selon l'une quelconque des revendications 8 à 11, dans lequel la seconde unité de source de lumière est une source de lumière laser.

13. Appareil formant endoscope selon la revendication 12,
dans lequel une longueur d'onde de lumière émise à partir de la seconde unité de source de lumière est comprise dans une plage allant de 495 nm à 570 nm.

14. Appareil
